# EUROPEAN PATENT APPLICATION

(11) **EP 2 581 040 A1**
(43) Date of publication of application: **17.04.2013**
(21) Application number: 11184497.3
(22) Date of filing: 10.10.2011
(51) Int. Cl.: A61B 5/145

(54) **TnT based cardiac hypertrophy risk related physiological training and guidance in athletes**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Zdunek, Dietmar, 82327 Tutzing (DE); Horsch, Andrea, 68259 Mannheim (DE)
(74) Representative: Dick, Alexander

(57) **Abstract**

A method of guiding physical exercise in a subject who regularly participates in physical exercise. The method is based on the determination of the amount of a cardiac troponin in a sample obtained at rest from said subject. Moreover, there is a method for assessing in a subject, who regularly participates in exercise and who shows an elevated level of a cardiac Troponin, whether the elevated level of the cardiac Troponin is caused by physical exercise, or not. Further envisaged are kits and devices adapted to carry out the method.

## Description

The present invention relates to a method of guiding physical exercise in a subject who regularly participates in physical exercise. The method is based on the determination of the amount of a cardiac troponin in a sample obtained at rest from said subject. Moreover, the present invention envisages a method for assessing in a subject, who regularly participates in exercise and who shows an elevated level of a cardiac Troponin, whether the elevated level of the cardiac Troponin is caused by physical exercise, or not. Further envisaged by the present invention are kits and devices adapted to carry out the present invention.

Physical activity has been shown to be beneficial and to contribute to improved overall survival in healthy individuals, individuals with chronic diseases, persons with diabetes mellitus und in the elderly (Thompson P.D. et al, Circulation 2003: 107: 3109). In this context it has been clearly shown that inactive men and women who subsequently become more active improves over all survival (Thompson P.D. et al, Circulation P.D., 2003: 107: 3109). It has been documented that physical activity of more than 3 hours per week induces increase of myocardial mass (Fagard R.H., Heart 2003: 89: 7 - 21). In case of intense training of at least 5 - 6 h per week "athletes heart" may develop (Dickhuth H.H. et al: Dt Zeitschrift für Sportmedizin 2001: 6: 205 - 210). The athletes heart is believed a physiological adaptation to increased physiological load (Opie L.H. in From Cell to Circulation. Philadelphia, Lippincott Williams & Wilkins, 2004). Depending on the type of exercise eccentric hypertrophy (in case of endurance training) or concentric hypertrophy (in case of strength training) may develop associated with significant remodelling of the heart (Lauschke J et al. Clin Res Cardiol 2009: 98: 80 - 88). This remodelling is associated with a proportional increase in cell length and cell width (Lauschke J et al: Clin Res Cardiol 2009: 98: 80 - 88).

Physiological hypertrophy appears to be driven by insulin like growth factor 1 and growth hormone and is believed not to be associated with interstitial fibrosis however might be associated with a vascular and cardiomyocyte growth mismatch (Kehat I et al, Circulation 2010: 122: 2727 - 2735). It is well documented that young athletes are at increased risk of sudden death. The causes vary and include hypertrophic cardiomyopathy, myocarditis and other causes (Maron B.J. et al, NEJM 2003: 349: 1064). Current recommendations in "athletes screening" to prevent undesired complications include family history, personal history and physical examination and in case of subjects at increased risk ECG and blood pressure monitoring (Maron B.J. et al J Am Coll Cardiol 2005: 45: 1322; Fletcher G.F. et al Circulation 2001: 104: 1694). So far specific laboratory tests have not been included into such an analysis (Hamm CW, Bassand JP, Agewall S, et al. ESC Guidelines for the management of acute coronary syndromes in patients presenting without persistent ST-segment elevation: The Task Force for the management of acute coronary syndromes (ACS) in patients presenting without persistent ST-segment elevation of the European Society of Cardiology (ESC). Eur Heart J 2011; DOI: 10.1093/eurheartj/ehr236).

Cardiac troponins T and I are the preferred biomarkers for the diagnosis of acute myocardial infarction (Anderson JL, ACC/AHA 2007 guidelines for the management of patients with unstable angina/non-ST-Elevation myocardial infarction. J Am Coll Cardiol. 2007;50(7):e1-e157). Moreover, there are several studies in which the relationship between the troponin level and mortality was analyzed. These studies show that increased levels of a cardiac troponin are associated with increased mortality (see e.g. Horwich et al., Circulation. 2003; 108: 833-838; Dierkes et al., Circulation. 2000; 102: 1964-1969;Waxmann et al., Journal of the American College of Cardiology Volume 48, Issue 9, 7 November 2006, Pages 1755-1762).

It has been recognized that elevated troponin levels may be detected in other clinical scenarios in which acute myocardial injury may occur as well as in several chronic disease states, including coronary artery disease, heart failure, and chronic kidney disease (see e.g. Omland et al.,N Engl J Med. 2009;361(26):2538-2547). Troponins T and I have also been shown to be detectable in individuals from the general population (see e.g. Wallace et al., Prevalence and determinants of troponin T elevation in the general population. Circulation. 2006;113(16):1958-1965).

Fortescue et al. (Ann Emerg Med. 2007 Feb;49(2): 137-43) determined cardiac Troponin levels in runners taking part at a marathon. Samples were taken 1 to 2 days before the race as wells as after the race. It was shown that Troponin increases are relatively common among marathon finishers and can reach levels typically diagnostic for acute myocardial infarction.

Whyte (Med Sci Sports Exerc. 2008 Aug;40(8): 1416-23) discloses that cardiac troponins are increased after endurance exercise in elite and recreational athletes. Whyte further discloses that endurance exercise may act as a promoter for right ventricular remodeling and a resultant trigger for ventricular arrhythmia.

Scharhag et al. (Med Sci Sports Exerc. 2008 Aug;40(8): 1408-15) discloses that cardiac biomarkers, e.g. cardiac troponins, can be increased after physical exercise. According to Scharhag, it would be still unclear whether exercise-associated increases of cardiac biomarkers represent a clinically relevant insult or are part of the physiological response to endurance exercise.

Recently, it was shown that higher levels of a cardiac Troponin measured with a highly sensitive Troponin assay are associated with left ventricular hypertrophy (see de Lemos et al., JAMA. 2010; 304(22):2503-2512). Thus, cardiac Troponins have been proposed as a marker for left ventricular hypertrophy.

The studies described by Whyte, Scharhag and Fortescue, however, are predominantly focused on the level of cardiac troponins after exercise. In the studies underlying the present invention the level of cardiac Troponin was determined in various groups of subjects at rest (at least 24 hours after physical exercise). Specifically, the level of Troponin T was determined in untrained subject and in subjects who regularly participate in exercise: in recreational athletes and elite athletes with an athlete's heart. Interestingly, it has been found that recreational athletes, i.e. less trained athletes without and athlete's heart, but with mild left ventricular hypertrophy had larger Troponin levels than more trained athletes with an athlete's hearts. The results obtained in the studies carried out in the context of the present invention are surprising since the prior art suggests that the level of cardiac Troponins would correlate to the degree of left ventricular hypertrophy. The studies show that the Troponin level allows for guiding physical exercise in subjects participating in physical exercise.

Since less trained, recreational athletes with mild left ventricular hypertrophy have increased Troponin T levels as compared to untrained subjects and elite athletes, it is important to monitor these athletes more carefully than untrained (healthy) subjects and elite athletes with an athlete's heart. In particular, athletes with significantly increased levels and of a cardiac troponin (indicating damage to the heart) shall reduce the intensity of physical exercise in order to reduce the troponin level.

Accordingly, the present invention relates to a method of guiding physical exercise in a subject who regularly participates in physical exercise, comprising
a) determining the amount of a cardiac troponin in a sample obtained at rest from said subject.

In a preferred embodiment the method further comprises the step of
b) comparing the amount of said cardiac troponin to a reference amount, thereby guiding physical exercise in said subject.

Thus, the present invention, in particular, relates to a method of guiding physical exercise in a subject who regularly participates in physical exercise, comprising the steps of
a) determining the amount of a cardiac troponin in a sample obtained at rest from said subject, and
b) comparing the amount of said cardiac troponin to a reference amount, wherein physical exercise is to be guided.

Preferably, physical exercise is guided by carrying out the further step of c) guiding physical exercise based on the result of the comparison carried out in step b).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented comparison and/or guidance based on said comparison in step (b).

The phrase "guiding physical exercise" as used herein, preferably, means assessing whether a subject as referred to herein can continue physical exercise (e.g. with the same or even with increased intensity), or whether the subject as referred herein cannot continue physical exercise (e.g. with the same intensity), and, thus shall reduce physical exercise (e.g. the intensity. Thus, by carrying out the method present invention, it can be assessed whether a subject is eligible to a continuation of physical exercise (e.g with the same intensity and/or with increased intensity), or whether subject is not eligible to a continuation of physical exercise (e.g. with the same intensity and/or with increased intensity). As will be understood by those skilled in the art, such an assessment is usually not intended to be correct for 100% of the subjects to be diagnosed. The term, however, requires that the assessment is correct for a statistically significant portion of the subj ects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, preferably mammals, and, more preferably, humans. In particular, the subject is male.

Preferably, the subject does not suffer from ACS (acute coronary syndrome). In particular the subject to be tested shall not suffer from myocardial infarction.

It is known in the art that there are several possible non-acute causes of troponin elevation, in particular chronic disorders, dysfunctions or diseases which include renal dysfunction, heart failure, systemic hypertension, hypertrophic cardiomyopathy, coronary artery disease, and kidney failure. Preferably, the subject to be tested by the method of the present invention does not suffer from the aforementioned disorders, dysfunctions or diseases. In particular, the subject to be tested shall not suffer from systemic hypertension. Further, the subject, preferably, does not suffer from tachy- or bradyarrhythmias, pulmonary embolism, severe pulmonary hypertension, acute neurological diseases, including stroke, aortic dissection, hypothyroidism, apical ballooning syndrome, infiltrative diseases, sepsis (which shall be clear, since some of the disease do not allow for participating in physical exercise). Accordingly, the subject is preferably apparently healthy with respect to cardiac disease. A subject who is apparently healthy with respect to cardiac disease, preferably, does not show symptoms of cardiac disease.

Preferably, the subject to be tested in accordance with the method of the present invention shall exhibit left ventricular hypertrophy. The term "left ventricular hypertrophy" is well known in the art. A detailed overview on left ventricular hypertrophy can be found in standard text books (see Swamy Curr Cardiol Rep (2010) 12:277-282). The term "left ventricular hypertrophy" (abbreviated "LVH") as used herein, preferably, relates to a thickening of the walls of the ventricles. It is well known in the art that left ventricular hypertrophy is a common type of cardiac remodelling that occurs when the heart experiences elevated workload. In particular, the heart and individual myocytes enlarge as a means of reducing ventricular wall and septal stress when face with increased workload or injury. Left ventricular hypertrophy can be classified as "pathological" and as "physiological". Physiological LVH occurs in healthy individuals after exercise. Pathological LVH results from pressure, or volume overload or from myocardial infarction.

In the context of the present invention it is particularly envisaged that the subject exhibits physiological left ventricular hypertrophy. Thus, the left ventricular hypertrophy in the context with the aforementioned method, preferably, shall be caused by physical exercise. Accordingly, the subject does, preferably, not suffer from hypertrophic non-obstructive cardiomyopathy, hypertrophic obstructive cardiomyopathy, and/or pressure overload hypertrophy. Moreover, as set forth above, the subject in the context of the aforementioned method does preferably not suffer from systemic hypertension. Preferably, the left ventricular hypertrophy is eccentric left ventricular hypertrophy.

LVH can be detected by diagnostic measures well known in the art e.g. electrocardiography, echocardiography, or cardiac magnetic resonance imaging (MRI). Preferably, LVH is detected by echocardiography. Moreover, criteria for the diagnosis of LVH are well known in the art (Mancia et al., European Heart J. 2007, 28: 1462, or H.M. Conolly and J.K. Oh, Chapter 14, pages 227 ff in Braunwalds Heart disease, chapter 8). The diagnosis of LVH, preferably, includes measurements of the septum diameter, left ventricular posterial wall thickness and end diastolic diameter, with calculation of left ventricular mass according to formulae known in the art. Particularly preferred criteria for diagnosing LVH are e.g. disclosed in the guidelines (Mancia et al., European Heart J. 2007, 28: 1462). Preferably, the Cornell voltage criteria, the Cornell product criteria, the Sokolow-Lyon voltage criteria or the Romhilt-Estes point score system is/are used (Mancia et al., European Heart J. 2007, 28: 1462). Also preferably, the LVH is determined by echocardiography as described by Dickhuth et al. and Scharhag et al. (Dickhuth et al., 1983, J Sports Med. 4: 21-26; Sharhag et al., 2002, Journal of the American College of Cardiology, Vol. 40, No: 10: 1556-63)

It has been shown in the context of the studies described herein that subjects participating in exercise who have mild LVH have the highest cardiac Troponin levels. Therefore, the subject, preferably, exhibits mild left ventricular hypertrophy, in particular mild physiological left ventricular hypertrophy. Preferably, a subject exhibiting mild left ventricular has a heart volume in the range between 11 to 12.9 ml per kg body weight. More preferably, said subject has a heart volume in the range between 11.5 to 12.9 ml per kg body weight. Even more preferably, said subject has a heart volume in the range between 11.8 to 12.5 ml per kg body weight. Most preferably, said subject has a heart volume in the range between 11.8 to 12.2 ml per kg body weight.

It was shown in the studies described herein that subjects who have an athlete's heart had lower Troponin levels than subjects with mild left ventricular hypertrophy. Therefore, the subject exhibiting mild left ventricular hypertrophy does, preferably, not have an athlete's heart. In particular, the subject exhibiting mild left ventricular hypertrophy shall have a heart volume of lower than 13 ml per kg body weight.

The subject in the context of the present invention shall participate in exercise. The term "exercise" as used herein, preferably, refers to any physical activity which is performed repeatedly over prolonged periods of time. Preferably, the exercise enhances or maintains physical fitness. The term "exercise", preferably, refers to strength exercise, and, more preferably, to endurance exercise. The term also includes a combination of endurance and strength exercise.

The terms "endurance exercise" and "strength exercise" are well known in the art. Whereas endurance exercise aims to increase cardiovascular endurance, strength exercise aims to increase short-term muscle strength. Preferred endurance exercises include cycling, swimming, rope, rowing, running, playing tennis and triathlon. Preferred strength exercises include weight training, eccentric training and sprinting. Also envisaged are combinations for the aforementioned exercises.

The subject in the context of the present invention shall, preferably, regularly participate in exercise. Accordingly, the subject shall, preferably, participate in exercise within a range of 60 minutes to 300 minutes per week. More preferably, the subject shall participate in exercise within a range of 120 minutes to 240, or even more preferably, within a range of 180 minutes to 300 minutes per week. Most preferably, the subject shall participate in exercise within a range of 180 minutes to 240 minutes per week. It was shown in the context of the invention that subjects participating in physical exercise within the aforementioned ranges had mild LVH. Surprisingly, these subjects had the largest Troponin levels.

The exercise may be split into various exercise units. The duration of a single exercise unit is, preferably, at least 30 minutes, and, more preferably, at least 45 minutes. Moreover, it is further envisaged that the exercise is interval exercise or continuous exercise.

Preferably, the exercise intensity, in particular the average exercise intensity, is at least 50% of the anaerobic threshold, more preferably, at least 60%, or most preferably at least 80% of the anaerobic threshold.

The term "anaerobic threshold" is well known in the art. Preferably, the anaerobic threshold is the point above which the muscles derive their energy from nonoxygenic sources rather than oxygenic sources during exercise. It is well known in the art that above the anaerobic threshold lactate (i.e. lactic acid) is produced faster than it is metabolized in the body. Accordingly, lactate accumulates in the blood when the exercise intensity is above the anaerobic threshold. When the exercise intensity is below the anaerobic threshold, lactate does not accumulate in the body. The anaerobic threshold can be determined by well known methods, e.g. by measuring lactate threshold in blood samples obtained during a ramp test where the exercise intensity is progressively increased. The anaerobic threshold is reached when lactic acid starts to accumulate. The threshold can also be performed non-invasively using gas-exchange methods in which the composition of the air inspired and expired is determined. Preferably, the anaerobic threshold is determined by a stepwise exercise test on a cycle ergometer using the method of Stegmann (H. Stegmann, W. Kindermann, A. Schnabel, Lactate Kinetics and Individual Anaerobic Threshold, Int J Sports Med 1981; 02(3): 160-165), which is herewith incorporated by reference with respect to its entire disclosure content. The method is, preferably, based on determining the gas exchange and the maximal oxygen consumption.

It is well known in the art that using performance-enhancing drugs in sport may increase septum thickness, and, thus, is associated with LVH, see e.g. Krieg et al. (Int J. Sports Med 2007: 28: 638 - 643). In the study described by Krieg the hypertrophy index was shown to be higher in power lifters who took anabolic steroids than in those who did not. Moreover users of anabolic drugs had evidence of diastolic dysfunction while non users had not. Thus, in a preferred embodiment of the method of the present invention the subject (in particular a subject with mild LVH) uses performance-enhancing drugs. In particular, said subject uses testosterone, anabolic steroids, insulin-like growth factor, and/or growth hormones.

In accordance with the planned embodiment, it can be assessed whether a subject can continue with physical exercise or cannot continue with physical exercise. A subj ect who cannot continue physical exercise shall, preferably, reduce physical exercise. This can be, preferably, achieved quantitatively i.e. by reducing the overall time spent on physical exercise (e.g. per week) and/or by reducing the intensity of physical exercise. Preferably, a subject who cannot continue physical exercise shall reduce the overall time spent on physical exercise and/or by reducing the intensity of physical by at least 15%, more preferably, by at least 25% and, even more preferably, by at least 35%, and most preferably by at least 50%. Further envisaged is that the subject refrains from physical exercise completely. Physical exercise may be continued (or increased) after cardiac Troponin levels have dropped.

A subject who can continue with physical exercise can continue physical exercise with the same intensity and/or can spend the same time on physical exercise. Alternatively, the subject even increase the intensity and/or the time spent on physical exercise.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

In accordance with the method of the present invention it is particularly contemplated that the sample has been obtained from the subject at rest. Preferably, a sample is deemed to have been obtained at rest, if it has been obtained not during exercise. More preferably, a sample is deemed to have been obtained at rest if it has been obtained not earlier than 6 hours, or not earlier than 12 hours after exercise. Even more preferably, a sample that has been obtained at rest has been obtained not earlier than 24 hours after physical exercise. Most preferably, a sample that has been obtained at rest has been obtained not earlier than 48 hours after physical exercise. Obtaining the sample at rest will allow the assessment of the level of the cardiac Troponin unbiased by Troponin increases that may occur during or after physical exercise.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin AT-Pase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subj ects at various conditions, but further studies are required to understand their respective role and rate (Masson et al., Curr Heart Fail Rep (2010) 7:15-21).

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectec-tion methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Bio-technol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations. The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically pro-vides the desired assessment in a suitable output format. The said result may, preferably, serve as an aid for guiding physical exercise in a subject.

Based on the comparison of the amount determined in step a) and the reference amount, it shall be possible to assess whether can continue physical exercise with the same intensity (or with increased intensity), or not. Therefore, the reference amount is to be chosen so that either a difference or an identity in the compared amounts allows identifying those test subjects which belong into the group of subjects which can continue physical exercise with the same intensity, or not. The method allows either excluding (rule-out) or identifying (rule-in) a subject as a subject who can continue physical exercise with the same intensity or not. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

The term "reference amount" as used herein refers to an amount which allows for allocation of a subject into either (i) the group of subjects being capable of continuing physical exercise with the same intensity or (ii) the group of subjects not being capable of continuing physical exercise with the same intensity. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom. Dependent on a desired sensitivity and specificity for a diagnostic method, the ROC plot allows deriving suitable thresholds.

Preferably, the reference amount is derived from a subject or group of subjects who regularly participate(s) in physical exercise, wherein said subject or subjects is/are known to be eligible to a continuation of physical exercise (e.g. with the same intensity) and/or from a subject or group of subjects who regularly participate(s) in physical exercise, wherein said subject or subjects is/are known not to be eligible to a continuation of physical exercise (e.g. with the same intensity and/or with increased intensity).

The following, preferably, applies as diagnostic algorithm.
- an identical amount, or, in particular, an essentially identical amount of the cardiac Troponin, or an amount of the cardiac troponin which is decreased as compared to the reference, indicates that the subject can continue physical exercise (e.g. with the same intensity), if the reference amount is derived from a subject or group of subjects who regularly participate(s) in physical exercise, wherein said subject or subjects known to be eligible to a continuation of physical exercise (e.g. with the same intensity), and/or
- an identical, in particular an essentially identical amount of the cardiac Troponin, or an amount of the cardiac troponin which is increased as compared to the reference indicates that the subject cannot continue physical exercise (e.g. with the same intensity), if the reference amount is derived from a subject or group of subjects who regularly participate(s) in physical exercise, wherein said subject or subjects are known not to be eligible to a continuation of physical exercise (e.g. with the same intensity and/or with increased intensity). Thus, the subject shall reduce physical exercise.

Preferred reference amounts derived from a subj ect, or from a group of subj ects who regularly participate(s) in physical exercise, wherein said subject or subjects are known to be eligible to a continuation of physical exercise (e.g. with the same intensity and/or with increased intensity) are, preferably, about 3.2 pg/ml, about 4.4 pg/ml or, more preferably, about 5.1 pg/ml.

Preferred reference amounts derived from a subject or a group of subjects who regularly participate(s) in physical exercise, wherein said subject or subjects are known not to be eligible to a continuation of physical exercise (e.g. with the same intensity and/or with increased intensity), are, in increasing order of preference about 5.2 pg/ml, about 6.5 pg/ml, or about 8.8 pg/ml. Further envisaged is that said reference amount is an amount of about 15 pg/ml.

Further, the reference amount may define a threshold amount, whereby an amount of Troponin in the sample of the test subject lower than the respective threshold shall be indicative for a subject being capable of continuing physical exercise with the same intensity, while an amount of Troponin in the sample of the test subject larger than the respective threshold shall be indicative for a subject being not capable of continuing physical exercise with the same intensity. Preferred reference amount defining a threshold amount for a cardiac Troponin as referred to in accordance with the present invention are, in increasing order of preference about 5.2 pg/ml, about 6.5 pg/ml, or about 8.8 pg/ml. Further envisaged is that the treshold amount is an amount of about 15 pg/ml.

The term "about" as used herein, preferably, means +/- 20%, +/- 10%, +/- 5%, +- 2 % or +-/ 1%, or +/-0% from the specific values referred to above.

In a preferred embodiment of the aforementioned method, the method further comprises of recommending a cardiac diagnostic measure, if the subject is not eligible to a continuation of physical exercise with the same intensity and/or with increased intensity. A preferred diagnostic measure is selected from the group consisting of is selected from the group consisting of Holter ECG, echochardiography, stress testing, CT scan and MRI scan

The definitions and explanations given herein above apply mutatis mutandis to the following (except if stated otherwise).

Moreover, the present invention relates to a method for assessing in a subject, who regularly participates in exercise and who has, preferably at rest, an elevated level of a cardiac Troponin, whether the elevated level of the cardiac Troponin is caused by physical exercise, or not, said method comprising a) determining the amount of a natriuretic peptide and/or of Growth Differentiation factor 15 (GDF-15) in a sample obtained from said sub-j ect.

In a preferred embodiment of the aforementioned method further comprises the step of b) comparing the amount of a natriuretic peptide and/or of Growth Differentiation factor 15 (GDF-15) to a reference amount. Thereby, it shall be assessed whether the elevated level of the cardiac Troponin is caused by physical exercise, or not.

Accordingly, the present invention relates, in particular, to a method for assessing in a subject, who regularly participates in exercise and who has, preferably at rest, an elevated level of a cardiac Troponin, whether the elevated level of the cardiac Troponin is caused by physical exercise, or not, said method comprising the steps of
a) determining the amount of a natriuretic peptide and/or of Growth Differentiation factor 15 (GDF-15) in a sample obtained from said subject, and
b) comparing the amount of a natriuretic peptide and/or of Growth Differentiation factor 15 (GDF-15) to a reference amount, whereby it is assessed whether the elevated level of the cardiac Troponin is caused by physical exercise, or not

As already explained elsewhere herein, athletes may have significantly increased troponin levels. The increased levels may be caused by physical exercise, and, thus, may be physiological. These physiological increased increases may be harmless since they may be decreased e.g. by reducing the extent of physical exercise. However, increased troponin levels may have other causes than physical exercise. In particular they may be of pathological origin. E.g. as described elsewhere herein, disorders or diseases affecting the heart may result in increased troponin levels. Troponin increases of pathological origin, however, usually require intervention (e.g. administration of drugs). The present invention allows to assess whether increased levels or a cardiac troponin are caused by physical exercise, or not.

Preferably, it is assessed whether the elevated levels of a cardiac Troponin are caused by physical exercise, or not by carrying out the further step of assessing whether the elevated levels of a cardiac Troponin are caused by physical exercise, or not.

In the context of the aforementioned method it shall be assessed in a subject who has an elevated level of a cardiac Troponin and who regularly participates in physical exercise whether the elevated level is caused by said physical exercise, or not. If the elevated level of a cardiac Troponin is not caused by physical exercise, they are usually pathological (and, thus, indicate cardiac disorders/disease, e.g. they are due to systemic hypertension) indicating that the subject needs to be closely monitored with respect to the cardiac condition. Preferably, the increased levels are caused by systemic hypertension, unrecognized hypertrophic cardiomyopathy (in particular in subject younger than 40 years of age), unrecognized coronary artery disease, and/or by heart failure (in particular in subjects older than 40 years).

As will be understood by those skilled in the art, such an assessment is usually not intended to be correct for 100% of the subj ects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein in the context with the aforementioned method relates to animals, preferably mammals, and, more preferably, humans. In particular, the subject is male. Preferably, the subject does not suffer from ACS (acute coronary syndrome), in particular the subject does preferably not suffer from myocardial infarction. Moreover, the subject, preferably, does not suffer from kidney failure and/or renal dysfunction.

Preferably, the subject in accordance with the method of the present invention shall exhibit left ventricular hypertrophy, in particular mild left ventricular hypertrophy. Moreover, the subject exhibiting mild left ventricular hypertrophy does not have an athlete's heart. The terms "left ventricular hypertrophy", "mild left ventricular hypertrophy" and "athlete's heart" have been described elsewhere herein. The definitions apply accordingly. The subject in the context of the aforementioned method, however may also exhibit pathological LVH. Accordingly, the subject may suffer from hypertrophic non-obstructive cardiomyopathy, hypertrophic obstructive cardiomyopathy, and/or pressure overload hypertrophy. Moreover, as set forth above, the subject in the context of the aforementioned method may suffer from systemic hypertension.

Moreover, the subject shall participate in exercise, in particular, in regular exercise as described elsewhere herein.

The subject shall have an elevated level of a cardiac Troponin, in particular in a serum or plasma sample. Preferably, the subject has a level of a cardiac troponin in a range from about 4 to 20 pg/ml, or, more preferably from about 6 to 15 pg/ml, in particular in a serum or plasma sample. Further preferred elevated cardiac Troponin levels are, in increasing order of preference 5.2 pg/ml, about 6.5 pg/ml, or about 8.8 pg/ml. Further envisaged is that said reference amount is an amount of about 15 pg/ml. Preferably, the aforementioned levels are Troponin T levels. Preferably, said levels are based on/established by the Troponin T test as described in Giannitsis et al. (Giannitsis E, Kurz K, Hallermayer K, Jarausch J, Jaffe AS, Katus HA. Analytical Validation of a High-Sensitivity Cardiac Troponin T Assay. Clin Chem. 56(2) 254-261, 2009) which herewith is incorporated by reference with respect to its entire disclosure content.

The term sample has been obtained elsewhere herein. As set forth above in the context of the method for guiding exercise, the sample to be analyzed in the context of the aforementioned method shall be obtained from the subj ect to be tested at rest.

The term "Growth-Differentiation Factor-15" or "GDF-15" relates to a polypeptide being a member of the transforming growth factor (TGF) cytokine superfamily. The terms polypeptide, peptide and protein are used interchangeable throughout this specification. GDF-15 was originally cloned as macrophage-inhibitory cytokine 1 and later also identified as placental transforming growth factor-15, placental bone morphogenetic protein, non-steroidal anti-inflammatory drug-activated gene 1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF -related cytokines, GDF-15 is synthesized as an inactive precursor protein, which undergoes disulfide-linked homodimerization. Upon proteolytic cleavage of the N terminal pro-peptide, GDF-15 is secreted as a ~28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific GDF-15 polypeptides, preferably with the amino acid sequence of human GDF-15, more preferably over the entire length of the specific GDF-15, e.g. of human GDF-15. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferably, natriuretic peptides according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolized in the blood, whereas NT-proBNP circulates in the blood as an intact molecule and as such is eliminated renally. The in-vivo half-life of NT-proBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical to human NT-proBNP, preferably over the entire length of human NT-proBNP. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of human NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Lab Invest 230:177-181), Yeo et al. (Yeo 2003, Clinica Chimica Acta 338:107-115). Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label, to the peptide.

In a preferred embodiment of the aforementioned method, the method further comprises the step of recommending a cardiac diagnostic method, if the elevated level of a cardiac Troponin are not caused by physical exercise. Preferably, the cardiac diagnostic method is selected from the group consisting of ECG, echocardiography, stress testing, Holter ECG, CT scan and MRI scan. Particularly preferred are ECG and echocardiography.

The term "reference amount" is defined elsewhere herein. The reference amount in the context with the aforementioned method is preferably derived from a sample of a (reference) subject, or from samples of group of (reference) subjects. The sample shall have been obtained at rest. The reference subject(s) shall regularly participate in physical exercise and may exhibit LVH (as the test subject). Moreover, the reference subject shall have an elevated level of a cardiac Troponin, preferably, of Troponin T (in particular at rest).

Preferably, the reference amount is derived from a subject or group of subjects wherein said subject or subjects is/are known i) to have (in particular at rest) elevated cardiac Troponin levels caused by physical exercise, or ii) to have (in particular at rest)elevated cardiac Troponin levels not caused by physical exercise.

The following, preferably, applies as diagnostic algorithm.
• an identical amount, or, in particular, an essentially identical amount of the natriuretic peptide and/or of GDF-15, or an amount of the natriuretic peptide and/or of GDF-15 which is (are) decreased as compared to the reference amount(s) of the natriuretic peptide and/or of GDF-15, indicates that the elevated level of a cardiac Troponin is caused by physical exercise, if the reference amount is derived from a subject (subjects) as set forth in i) above and/or
• an identical amount, or, in particular, an essentially identical amount of the natriuretic peptide and/or of GDF-15, or an amount of the natriuretic peptide and/or of GDF-15 which is (are) decreased as compared to the reference amount(s) of the natriuretic peptide and/or of GDF-15, indicates the elevated level of a cardiac Troponin is not caused by physical exercise, if the reference amount is derived from a subject (subjects) as set forth in ii) above

Preferred reference amounts for GDF-15 derived from a subject, or from a group of subjects as set forth in i) above are in increasing order of preference amounts of about 300 pg/ml, about 350 pg/ml, or about 400 pg/ml.

Preferred reference amounts for GDF-15 derived from a subject, or from a group of subjects as set forth in ii) above are in increasing order of preference amounts of about 450 pg/ml, about 500 pg/ml, or about 550 pg/ml.

Preferred reference amounts for a natriuretic peptide, in particular for NT-proBNP, derived from a subject, or from a group of subjects as set forth in i) above are in increasing order of preference amounts of about 20 pg/ml, or about 25 pg/ml.

Preferred reference amounts for a natriuretic peptide, in particular for NT-proBNP, derived from a subject, or from a group of subjects as set forth in ii) above are in increasing order of preference amounts of about 35 pg/ml, about 50 pg/ml, or about 60 pg/ml.

Further, the reference amount may define a threshold amount, whereby an amount of GDF-15 and/or of the natriuretic in the sample of the test subject lower than the respective threshold amount indicates that the elevated troponin level is caused by physical exercise, and/or whereby an amount of GDF-15 and/or of the natriuretic in the sample of the test subject larger than the respective threshold amount indicates that the elevated troponin level is not caused by physical exercise.

In a preferred embodiment of the aforementioned method, the method recommending a cardiac diagnostic method, if the elevated level of a cardiac Troponin are not caused by physical exercise. This will allow for assessing the cause of Troponin elevation. Preferably, the recommended diagnostic method is selected from the group consisting of Holter ECG, echocardiography, stress testing, CT scan and MRI scan. Moreover, genetic testing for hypertrophic cardiomyopathy may be recommended.

Moreover, the present invention relates to the use of a cardiac Troponin and/or of a detection agent, which specifically binds thereto, in a sample of a subject who regularly participates in physical exercise for guiding physical exercise in said subject.

Also envisaged by the present invention is the use of GDF-15 or of a detection agent, which specifically binds thereto, and/or of a natriuretic peptide, or of a detection agent which specifically binds thereto in a sample of a subject who regularly participates in physical exercise and who has an elevated level of a cardiac Troponin for assessing whether the elevated level of the cardiac Troponin is caused by physical exercise.

The term "detection agent" as used herein refers to an agent which is capable of specifically recognizing and binding the biomarker referred to herein (a cardiac Troponin, GDF-15, or a natriuretic peptide, in particular to NT-proBNP) when present in a sample. Moreover, said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. Antibodies as referred to herein include both polyclonal and monoclonal antibodies, as well as fragments thereof, such as Fv, Fab and F(ab)₂ fragments that are capable of binding antigen or hapten. Also envisaged are single chain antibodies and humanized hybrid antibodies wherein amino acid sequences of a non-human donor antibody exhibiting a desired antigen-specificity are combined with sequences of a human acceptor antibody.

The present invention further relates to a device for guiding physical exercise in a subject who regularly participates in physical exercise, said device comprising:
a) an analyzing unit comprising a detection agent for a cardiac Troponin which allows for the determination of the amount of said cardiac Troponin; and
b) an evaluation unit comprising a data processor having implemented an algorithm for comparing the amounts determined by the analyzing unit with reference amount(s) stored in a database in order to establish the guidance by indicating whether the subject can continue with physical exercise, or not, wherein the reference amount is derived from a sample from a reference subject as described herein elsewhere in the context with the method of guiding physical exercise, and the algorithm is an algorithm as defined in the context with the method of guiding physical exercise.

The present invention further relates to a device for assessing whether an elevated Troponin level in a subject is caused by physical exercise or not, said device comprising:
a) an analyzing unit comprising a detection agent for a natriuretic peptide and/or GDF 15 which allows for the determination of the amount of said natriuretic peptide and/or GDF-15; and
b) an evaluation unit comprising a data processor having implemented an algorithm for comparing the amounts determined by the analyzing unit with reference amount(s) for a natriuretic peptide and/or GDF-15 stored in a database in order to establish the assessment by indicating whether the elevated Troponin level in a subject is caused by physical exercise or not, wherein the reference amount is derived from a sample from a reference subject as described herein elsewhere in the context with the method of assessing whether an elevated Troponin level in a subject is caused by physical exercise, and the algorithm is an algorithm as defined in the context with the said method.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The diagnostic results may be given as output of parametric diagnostic raw data, preferably, as absolute or relative amounts. It is to be understood that these data may need interpretation by the clinician. However, also envisage are expert system devices wherein the output comprises processed diagnostic raw data the interpretation of which does not require a specialized clinician. Preferably, the device of the present invention can be used to carry out the aforementioned method of the present invention in an automated manner.

The present invention further encompasses a kit for guiding physical exercise in a subject who regularly participates in physical exercise, said kit comprising a detection agent for a cardiac Troponin and a standard which reflect the reference amount as derived from a sample from a subject known to be capable of continuing physical exercise or derived from a sample from a subject known not to be capable of continuing physical exercise.

The present invention, finally, encompasses a kit for assessing whether an elevated Troponin level in a subject is caused by physical exercise or not, said kit comprising a detection agent for a cardiac Troponin and a standard which reflect the reference amount as derived from a sample from a subject having an elevated cardiac Troponin, said being known to have elevated Troponin levels caused by physical exercise or as derived from a sample from a subject having an elevated cardiac Troponin, said being known to have elevated Troponin levels not caused by physical exercise.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards which reflect the reference amounts as described and referred to elsewhere herein in detail. The detection agent is, preferably, immobilized on a carrier, and, preferably, a test stripe.

The following Examples shall merely illustrate the invention. They shall not be construed, whatsoever, to limit the scope of the invention.

### Example 1:

### Cohorts

A total of 1452 persons from the general population without apparent risk factors were included into the study. Persons with a history of coronary artery disease or peripheral artery disease were excluded, so were persons with documented diabetes mellitus. In addition persons meeting the criteria of systemic hypertension and the metabolic syndrome were excluded from the study. Another control group consisted of patients in whom all risk factor were excludes except systemic hypertension as confirmed by medical history, drug therapy of hypertension of blood pressure , measured at least three times exceeding 90 mmHg diastolic and/or 140 mm Hg systolic. (n = 550)

In addition 11 male athlethes with an athletes heart as documented with a heart volume of at least 13 ml/kg bodyweight (Median 14.1 mg/kg) a and 10 male athletes with a heart volume below 13 ml/kg bodyweight (Median 12.0 ml/kg) were included in the study. As control 9 apparently healthy subjects (Median 10.3 mg/kg, less than 2 hours physical exercise per week) were included into the study. Heart disease was excluded using ECG at rest and after exercise according to standard criteria, in addition a echocardiogram was without abnormalities.

### Determination of Troponin T, GDF-15 and NT-proBNP

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids (analytical sensitivity below 1,0).

NT-proBNP was determined using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

To determine the concentration of GDF-15 in serum and plasma samples, an Elecsys prototype test was employed, using a polyclonal, GDF-15 affinity chromatography-purified, goat anti-human GDF-15 IgG antibody from R&D Systems (AF957). In each experiment, a standard curve was generated with recombinant human GDF-15 from R&D Systems (957-GD/CF). The results with new batches or recombinant GDF-15 protein were tested in standard plasma samples and any deviation above 10% was corrected by introducing an adjustment factor for this assay. GDF-15 measurements in serum and plasma samples from the same patient yielded virtually identical results after correction for eventual dilution factors. The detection limit of the assay was 200 pg/ml.

All persons were tested at rest, in athletes the last exercise occurred at least 24 h before blood was drawn. Blood was centrifuged within 30 minutes and the resulting serum was kept at least - 20 degrees Celsius until tested.

### Example 3: Results

The results are summarized in the following Table (indicated are the medians, and the 25. and 75. percentiles; AH: Athlete's heart):

| | Troponin T | NT-pro BNP | GDF 15 |
|---|---|---|---|
| | pg/ml | pg/ml | pg/ml |
| Apparently healthy | 1,2 | 45,4 | 502 |
| | 0,1 - 3,4 | 22-81 | 395 - 676 |
| Systemic hypertension | 3,0 | 69,6 | 603 |
| | 0,7 - 5,9 | 28 - 126 | 458 - 806 |
| Physically | 2,7 | 21,0 | 375 |
| Active subjects | 1,7 - 3,6 | 6-33 | 346-417 |
| Athletes | 6,5 | 17,0 | 349 |
| | 4,4-8,8 | 13,5 - 21 | 312 - 366 |
| Athletes with AH | 4,0 | 19 | 397 |
| | 3,2 - 5,2 | 9-22 | 350 - 467 |

The highest determined level was 14.6 pg/ml.

**Table 2 summarises the cardiac parameter of the different athletes groups:**

| | Phy active subj | Athletes | Athletes with AH |
|---|---|---|---|
| Heart Vol abs ml | 795 | 921 | 1011 |
| | 776 - 855 | 909 - 949 | 988 - 1070 |
| Heart Vol ml/Kg | 10,3 | 12,0 | 14,1 |
| | 10,2 - 10,8 | 11,8 - 12,5 | 13,6 - 14,6 |
| LVM abs | 161 | 183 | 204 |
| | 143 - 170 | 174-189 | 192 - 223 |
| LVM/BSA | 76 | 90 | 110 |
| | 73 - 87 | 88 - 95 | 100 - 112 |

| | | | |
|---|---|---|---|
| LVM: left ventricular mass, BSA: Body Surface Area | | | |

### Case studies

**Case 1:** A 26 year old male who is training for the national rowing championship for three quarters of a year with regular weight lifting and rowing on the lake in total approximately 7 hours a week returns for regular check up. His ECG is without abnormality, echocardiography reveals a heart volume of 1050 ml (14.2 ml/kg), a left ventricular mass/body surface area of 109, thus meeting the criteria of athletes heart. His serum troponin T is 3.1 pg/ml, his NT-pro BNP is 18 pg/ml and GDF 15 is 368 pg/ml. He is informed that his training program is adequate and no abnormalities can be found and that he can continue his program as is.

**Case 2:** A 18 year old male has recently decided to start a rigorous training programs as did his friends. He visits 5 times a week a sport studio where he does strength and endurance training. He sees his doctor as he considers anabolic drugs sold under the counter at his studio. On request he receives an ECG with no abnormalities, his troponin T is 9.6 pg/ml, NT-pro BNP is 21 pg/ml and his GDF-15 is 382 pg/ml. He is informed that his troponin T is too high which could be explained that his cardiac hypertrophy (heart volume 11.5 ml/kg bodyweight) developed too fast and he is advised to significantly reduce his training program. Six weeks later his troponin T has fallen to 6.1 pg/ml while GDF 15 and NT-pro BNP remained unchanged.

Case 3: A 23 year old male who regularly visits the sport studio for approximately 3 hours a week notes extrabeats of his heart and he decides to see his doctor. The ECG is normal an his blood pressure is 130/90 and thus borderline. His troponin T is 5.2 pg/ml, his NT-pro BNP was 71 pg/ml and his GDF 15 was 693 pg/ml. He is informed that NT-pro BNP and GDF 15 levels are higher than expected. The patient is recommended a 24 h blood pressure test which confirmed nocturnal systemic hypertension which was then treated.

### Conclusion:

Physical exercise is known to be beneficial and to prolong life, extended exercise is also known to induce left ventricular hypertrophy, this may be associated with adverse events such as acute cardiac death, arrhythmia and cardiac fibrosis, in addition under vigorous exercise rapid cardiac hypertrophy may proceed faster that angiogenic growth resulting in a mismatch associated with reduced oxygen supply to the cardiac muscle. Surprisingly it has been found, that individuals with regular exercise have increased troponin T levels as compared to the general population and as compared to elite athletes. Increased Troponin levels are associated with increased cardiac death. In addition the level of cardiac troponin T did not correlate with the extent of cardiac hypertrophy indicating that troponin T concentrations are related to type of exercise. This gives the basis for using troponin T levels to direct exercise.

Another aspect of the inventions is that exercise induced cardiac hypertrophy may render recognition of pathological conditions more difficult such as masked hypertension, hypertrophic cardiomyopathy in younger individuals and chronic artery disease or asymptomatic heart failure in elder individuals. In this case the derterination of NT-pro BNP and GDF 15 aids in dissecting physiological and pathological troponin T elevations. The suspected cause of troponin T elevation then triggers additional diagnostic procedures such an echocardiography, 24 ECG reading, genetic testing etc.

In summary, the current invention represents an important tool to make exercise safer and to recognise more reliably early pathological conditions in individuals with regular exercise.

## Claims

1. A method of guiding physical exercise in a subject who regularly participates in physical exercise, comprising determining the amount of a cardiac troponin in a sample obtained at rest from said subject.

2. The method of claim 1, further comprising the step of comparing the amount of said cardiac troponin to a reference amount, thereby guiding physical exercise.

3. The method of claims 1 and 2, wherein the subject exhibits mild left ventricular hypertrophy.

4. The method of any one claims 1 to 3, wherein the subject exhibiting mild left ventricular hypertrophy has a heart volume in the range from 11.0 to 12.9 ml/kg body weight.

5. The method of any one of claims 1 to 4, wherein the subject participates in exercise within a range of 60 minutes to 300 minutes per week.

6. The method according to any of claims 1 to 5, wherein the subject participates in endurance exercise and/or strength exercise.

7. The method of any one of claims 1 to 6, wherein the subject is human.

8. The method of any one of claims 1 to 7, wherein
i) the reference amount is derived from a subject or group of subjects who regularly participate(s) in physical exercise, wherein said subject or subjects is/are known to be eligible to a continuation of physical exercise, wherein an essentially identical amount of the cardiac Troponin, or an amount of the cardiac troponin which is decreased as compared to the reference, indicates that the subject can continue physical exercise, and/or
ii) the reference amount is derived from a subject or group of subjects who regularly participate(s) in physical exercise, wherein said subject or subjects is/are known not to be eligible to a continuation of physical exercise, wherein an essentially identical amount of the cardiac Troponin, or an amount of the cardiac troponin which is increased as compared to the reference, indicates that the subject cannot continue physical exercise.

9. A method for assessing in a subject, who regularly participates in exercise and who has, preferably at rest, an elevated level of a cardiac Troponin, whether the elevated level of the cardiac Troponin is caused by physical exercise, or not, said method comprising the steps of
a) determining the amount of a natriuretic peptide and/or of Growth Differentiation factor 15 (GDF-15) in a sample obtained from said subject, and optionally
b) comparing the amount of a natriuretic peptide and/or of Growth Differentiation factor 15 (GDF-15) to a reference amount, whereby it is assessed whehter the elevated Troponin level is caused by physical exercise, or not.

10. The method of claim 9, wherein the subject has a level of a cardiac troponin, in particular of cardiac Troponin T, in a range from 4 to 20 pg/ml, in particular in a serum or plasma sample.

11. The method of any one of claims 9 and 10, further comprising the step of recommending a cardiac diagnostic method, if the elevated level of a cardiac Troponin is not caused by physical exercise.

12. The method of claim 11, wherein the diagnostic method is selected from the group consisting of Holter ECG, echochardiography, stress testing, 24 h ECG, CT scan and MRI scan.

13. Use of a cardiac Troponin and/or of a detection agent, which specifically binds thereto, in a sample of a subject who regularly participates in physical exercise for guiding physical exercise in said subject.

14. Use of GDF-15 and/or of a natriuretic peptide, or of a detection agent, which specifically binds thereto, in a sample of a subject who regularly participates in physical exercise and who has an elevated level of a cardiac Troponin for assessing whether the elevated level of the cardiac Troponin is caused by physical exercise, or not.

15. A device for guiding physical exercise in a subject who regularly participates in physical exercise, said device comprising:
a) an analyzing unit comprising a detection agent for a cardiac Troponin which allows for the determination of the amount of said cardiac Troponin; and
b) an evaluation unit comprising a data processor having implemented an algorithm for comparing the amount determined by the analyzing unit with reference amount(s) stored in a database in order to establish the guidance by indicating whether the subject can continue with physical exercise, or not, wherein the reference amount is derived from a sample from a reference subject as defined in claim 8, and the algorithm is an algorithm as defined in claim 8.
